(19) Europäisches Patentamt
European Patent Office
Office européen des brevets

(11) Veröffentlichungsnummer: **0 039 857**
**A1**

# (12) EUROPÄISCHE PATENTANMELDUNG

(21) Anmeldenummer: **81103325.7**

(22) Anmeldetag: **02.05.81**

(51) Int. Cl.³: **A 61 K 7/48**
A 61 K 7/32, A 61 K 31/215
A 61 K 31/165, A 61 K 31/085
A 61 K 31/10

(30) Priorität: **12.05.80 DE 3018114**

(43) Veröffentlichungstag der Anmeldung:
**18.11.81 Patentblatt 81/46**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Möller, Hinrich, Dr.**
**Schumannstrasse 11**
**D-4019 Monheim(DE)**

(72) Erfinder: **Osberghaus, Rainer, Dr.**
**Südallee 47**
**D-4000 Düsseldorf-Urdenbach(DE)**

## (54) Hautpflegemittel.

(57) Die Mittel enthalten 0,5 bis 30 (3 bis 15) Gewichtsprozent an arylsubstituierten Alkansäurederivaten der allgemeinen Formel (I) als Inhibitor für bakterielle Lipase- und Esteraseenzyme, um Spaltungsreaktionen von Hauttriglyceriden mit Fettsäurefreisetzung zu verhindern. Solche Fettsäuren können Ursache für Hautausschläge sein.

$$Ar - (X)_p - (CR^1R^2)_m - (CR^3R^4)_n - COR^5 \qquad (I)$$

Ar = gegebenenfalls substituierter Arylrest
X = Sauerstoff oder Schwefel
$R^1$ = H, Alkyl-($C_{1-4}$) oder ggf. substituierter Phenylrest
$R^2$ = H oder -OH
$R^3$, $R^4$ = unabhängig voneinander H oder Alkyl-($C_{1-4}$)
$R^5$ = Alkoxy-($C_{1-10}$), $-NH_2$, Mono- oder Dialkylaminogruppe
p = 0 oder 1, m = 1 oder 2, n = 0, 1 oder 2
Die Alkansäurederivate können auch als Wirkstoffe in Deodorantien verwendet werden.

EP 0 039 857 A1

COMPLETE DOCUMENT

HENKEL KGaA
ZR-FE/Patente
Z/Ge

Patentanmeldung

D 6169 EP

"Hautpflegemittel"

Gegenstand der Erfindung sind Hautpflegemittel mit einem Gehalt an arylsubstituierten Alkansäurederivaten.

Es ist allgemein bekannt, daß unter den auf der Hautoberfläche vorhandenen Bakterien einige, wie zum Beispiel insbesondere Corynabacterium Acnes, Lipasen und Esterasen ausscheiden, die auf den Hauttalg und andere auf der Haut vorhandene Triglyceride einwirken und daraus Fettsäuren freisetzen. Diese, durch enzymatische Spaltungsreaktionen erhaltenen Fettsäuren, sind neben anderen Faktoren die Ursache für unangenehme und unästhetische Hautausschläge, die vor allem im Gesicht und auf dem oberen Teil des Thorax und Rückens auftreten. Auf Grund dieser Erkenntnisse sind bereits enzyminhibierende Substanzen in kosmetischen Zusammensetzungen zur Vermeidung dieser nachteiligen Erscheinungen und zur Erhaltung der Hautgesundheit eingesetzt worden, ohne daß bisher allgemeine durchgreifende Erfolge erzielt werden konnten. Es hat daher nicht an Versuchen gefehlt, weitere Substanzen aufzufinden, die sich zur Bekämpfung dieser Hautveränderungen in kosmetischen Zusammensetzungen einsetzen lassen.

Es wurde nun gefunden, daß Hautpflegemittel mit einem Gehalt an arylsubstituierten Alkansäurederivaten der allgemeinen Formel

$$Ar - (X)_p - (CR_1R_2)_m - (CR_3R_4)_n - COR_5$$

/2

in der Ar für einen gegebenenfalls substituierten Arylrest, X für Sauerstoff oder Schwefel, $R_1$ für Wasserstoff, einen niederen Alkylrest mit 1 bis 4 Kohlenstoffatomen oder einen gegebenenfalls substituierten Phenylrest, $R_2$ für Wasserstoff oder die OH-Gruppe, $R_3$ und $R_4$ unabhängig voneinander für Wasserstoff oder eine niedere Alkylgruppe mit 1 bis 4 Kohlenstoffatomen, $R_5$ für eine Alkoxygruppe mit 1 bis 10 Kohlenstoffatomen, eine $NH_2$-Gruppe, eine Mono- oder Dialkylaminogruppe, p für 0 oder 1, m für 1 oder 2 und n für 0, 1 oder 2 stehen, sich auf Grund von deren enzyminhibierenden Wirkungen gut zur Bekämpfung derartiger Hautveränderungen und zur Erhaltung der Hautgesundheit eignen.

Die oben definierten arylsubstituierten Alkansäurederivate können auch als Wirkstoffe in Deodorantien verwendet werden.

Die Herstellung der in den erfindungsgemäßen Hautpflegemitteln einzusetzenden arylsubstituierten Alkansäurederivate kann nach allgemein bekannten Verfahren der organischen Synthese erfolgen. Die Ester können leicht durch Reaktion entsprechender Alkansäuren mit den entsprechenden Alkoholen in Gegenwart von Schwefelsäure erhalten werden. Die Amide werden vorteilhaft über die entsprechenden Säurechloride hergestellt.

Als den erfindungsgemäß einzusetzenden arylsubstituierten Alkansäurederivaten zugrunde liegende Carbonsäuren sind zum Beispiel Phenyl-, o-Methoxyphenyl-, m-Methoxyphenyl-, p-Methoxyphenyl-, 3,4-Dimethoxyphenyl-, 3,4,5-Trimethoxyphenyl-, o-Chlorphenyl-, m-Chlorphenyl-, p-Chlorphenyl-, 2,4-Dichlorphenyl-, 2,5-Dichlorphenyl-, 3,4-Dichlorphenyl-, 3,5-Dichlorphenyl-, o-Hydroxyphenyl-, m-Hydroxyphenyl-, p-Hydroxyphenyl-essigsäure, -propionsäure, -buttersäure, -valeriansäure, 2,2-Diphenylessigsäure, 2,2-Diphenyl-, 3,3-Diphenyl-propionsäure, Benzilsäure, Phenoxy-essigsäure, -propionsäure, -buttersäure, -valeriansäure, o-Methoxy-, m-Methoxy-, p-Methoxy-, o-Chlor-, m-Chlor-, p-Chlor-, o-Hydroxy-, m-Hydroxy-, p-Hydroxy-, o-Methyl-, m-Methyl-, p-Methyl-, o-Amino-, m-Amino-, p-Amino-, o-Butoxy-, m-Butoxy-, p-Butoxy-, o-Benzyloxy-, m-Benzyloxy-, p-Benzyloxy-, 2,3-Dichlor-, 2,4-Dichlor- , 3,4-Dichlor-, 3,5-Dichlor-, 2,3-Dimethyl-, 2,4-Dimethyl-, 3,4-Dimethyl-, 3,5-Dimethyl-, 2,3-Dimethoxy-, 2,4-Dimethoxy-, 3,4-Dimethoxy-, 3,5-Dimethoxy-, 3,4-Methylendioxy-phenoxy-essigsäure, -propionsäure, -2-methylpropionsäure, -buttersäure, -valeriansäure, Phenylthio-, o-Chlorphenylthio-, m-Chlorphenylthio-, p-Chlorphenylthio-, α-Naphthoxy-, β-Naphthoxy-essigsäure, -propionsäure, -2-methylpropionsäure, -buttersäure, -valeriansäure anzuführen.

Als Alkylreste der erfindungsgemäß einzusetzenden esterförmigen arylsubstituierten Alkansäurederivate sind der Methyl-, Ethyl-, Propyl-, Isopropyl-, n-Butyl-, iso-Butyl-, sek.-Butyl-, tert.-Butyl-, Pentyl-, Hexyl-, 2-Ethylhexyl-, Octyl- und Decylrest zu nennen.

Als Amidkomponente der erfindungsgemäß einzusetzenden arylsubstituierten Alkansäurederivate kommen neben primären Amiden zum Beispiel Methyl-, Dimethyl-, Ethyl-, Diethyl-, Propyl-, Dipropyl-, Methylpropyl-, 2-Propyl-, Di-2-propyl-, Butyl-, Dibutyl-, 2-Butyl-, sek.-Butyl-, tert.-Butyl-, Hexyl-, Dihexyl-, 2-Ethylhexyl-, Octyl-, Ethyloctyl-, Decyl-, Dodecyl-, Ethanol-, Diisopropanol-, 3-Methoxypropyl-, 3-(2-Ethylhexoxy)-propyl-, Benzyl-amid, Anilid, N-Methylanilid, Piperidid, 2-Methyl-, 3-Methyl-, 4-Methyl-piperidid, 2,6-Dimethyl-, 3,5-Dimethyl-piperi-

/4

did, Morpholid und 2,6-Dimethylmorpholid in Frage.

Als in den erfindungsgemäßen Hautpflegemitteln einzusetzende arylsubstituierte Alkansäurederivate sind danach zum Beispiel p-Chlorphenylacet-diethylamid, Benzilsäureethylester, 3,4-Dimethoxy-phenylacet-, 3,3-Diphenyl-propionsäure-, 2-(p-Chlor-phenoxy)-2-methyl-propionsäure-, 2-(p-Chlor-phenylthio)-2-methyl-propionsäure-, p-Chlor-phenoxyacet-, 3,4-Dimethyl-phenoxyacetdiethylamid zu nennen.

Die erfindungsgemäßen Hautpflegemittel enthalten die als Wirkstoffe zur Erhaltung der Hautgesundheit und Bekämpfung genannter Hautveränderungen wie zum Beispiel Akne oder Seborrhoea oleosa dienenden arylsubstituierten Alkansäurederivate in einer Menge von 0,5 bis 30 Gewichtsprozent, vorzugsweise 3 bis 15 Gewichtsprozent, bezogen auf die gesamte Präparation.

Die erfindungsgemäßen Hautpflegemittel werden in Gestalt der für diese Zwecke üblichen Präparationen vorliegen, also Cremes, Gele, Lotionen, Hautmilch, Badeöle, Salben, Pomaden, Masken, Seifen und Aerosole. Ihre Herstellung erfolgt nach den für derartige Präparationen üblichen Verfahren. Dabei werden die als Wirkstoff dienenden arylsubstituierten Alkansäurederivate mit den für die Hautpflegemittel als Basismaterial dienenden organischen Substanzen wie Wachsen, Fetten, Ölen, Alkoholen, Mineralölen vermischt und danach mit den weiteren Bestandteilen der Hautpflegemittel wie Netzmitteln, Emulgatoren, Verdickungsmitteln, Konservierungsmitteln, Parfüms, Wasser, Lösungsmitteln, Treibgasen zum fertigen Endprodukt konfektioniert. Die Mengen der in den erfindungsgemäßen Hautpflegemitteln außer den arylsubstituierten Alkansäurederivaten vorhandenen Rezepturbestandteile bewegen sich in den für vergleichsweise Produkte üblichen Größenordnungen. /5

Die nachfolgenden Beispiele sollen den Gegenstand der Erfindung näher erläutern, ohne ihn jedoch hierauf zu beschränken.

## Beispiele

Zunächst wird die Herstellung der in den erfindungsgemäßen Hautpflegemitteln eingesetzten arylsubstituierten Alkansäurederivate beschrieben.

A) p-Chlorphenylacet-diethylamid

Zu einer von 45 g (0,615 Mol) Diethylamin in 250 ml Ether wurden unter Rühren und Kühlen bei Raumtemperatur 38,9 g (0,205 Mol) p-Chlorphenyl-acetylchlorid in 50 ml Ether getropft. Anschließend wurde das Reaktionsgemisch 1 Stunde bei Raumtemperatur und 1 Stunde bei Siedetemperatur gerührt, der Niederschlag abgesaugt, mit Ether nachgewaschen, die Etherphase mit verdünnter Salzsäure und Wasser gewaschen und der Ether abdestilliert. Der Eindampfrückstand wurde unter vermindertem Druck destilliert. Es wurden 33 g p-Chlor-phenylacet-diethylamid vom Siedepunkt 122° C bei 0,16 mbar und einem Brechungsindex $n_D^{20}$ 1,5344 erhalten.

In analoger Weise wurden die nachstehend aufgeführten Substanzen aus den entsprechenden Ausgangsmaterialien hergestellt.

B) 3,4-Dimethoxy-phenylacet-diethylamid

Kp. 144° C/0,08 mbar $n_D^{20}$

C) 3,3-Diphenyl-propionsäure-diethylamid.

Schmp. 71 bis 73° C

D) 2-(p-Chlorphenoxy)-2-methylpropionsäure-diethylamid

Schmp. 58 bis 61° C

E) 2-(p-Chlor-phenylthio)-2-methylpropionsäure-diethylamid

Kp. 114° C/0,01 mbar $n_D^{20}$ 1,5575

/7

F) p-Chlor-phenoxyacet-diethylamid
Kp. 135° C/0,20 mbar $n_D^{20}$ 1,5364

G) 3,4-Dimethyl-phenoxyacet-diethylamid
Kp. 131° C/0,20 $n_D^{20}$ 1,5240

H) Benzilsäure-ethylester +)
Schmp. 34° C, Kp. 201° C/28 mbar

+) Die Herstellung erfolgte gemäß Angaben in Beilstein 10 II, Seite 225.

Zur Feststellung der enzyminhibierenden Wirksamkeit wurde folgende Testmethodik verwendet. Es wurden jeweils 0,2 Mol in 10 bis 20 ml physiologischer Natriumchlorid-Lösung Substrat eingesetzt. Als Testenzyme dienten:

Esterase aus Schweineleber (Carboxylic-esterhydrolase EC 3.1.1.1)
Spezifische Aktivität: circa 100 U/mg, Einsatzkonzentration: 1 mg in 16 ml Lösung.
Als Substrat diente Citronensäuretriethylester.

Lipase aus Rhizopus arrhizus (Triacylglyceroacylhydrolase EC 3.1.1.3)
Spezifische Aktivität: circa 1 200 U/mg, Einsatzkonzentration: 0,25 mg in 20 ml Lösung
Als Substrat diente Olivenöl. Zur Stabilisierung der Emulsion wurde Natriumdesoxycholat und Gummi arabicum verwendet. Die Hemmwerte wurden bestimmt durch azidimetrischen Vergleich der Kontrolle mit der durch die Prüfsubstanz gehemmten Reaktion und in % ausgedrückt.
Als Konzentration für die Hemmsubstanzen wurden 2 500 ppm gewählt.

/8

Die bei den Testen festgestellten Hemmwerte sind nachstehender Tabelle 1 zu entnehmen.

Tabelle 1

| Produkt | Hemmwerte in % gegen Esterase | Lipase |
|---|---|---|
| A | 77 | 23 |
| B | 60 | 10 |
| C | 57 | 12 |
| D | 11 | 52 |
| E | 30 | 59 |
| F | 65 | 10 |
| G | 48 | 12 |
| H | 54 | 28 |

Zur näheren Erläuterung des Erfindungsgegenstandes werden nachstehend einige Beispiele für erfindungsgemäße Hautpflegemittel aufgeführt:

Hautpflegesalbe gegen Akne

| | |
|---|---|
| Gemisch aus höhermolekularem Fettalkohol | 30,0 Gewichtsteile |
| Wachsestern und mineral. Fetten (Amphocerin K(R)) | |
| Ölsäuredecylester | 10,0 Gewichtsteile |
| Produkt A | 10,0 Gewichtsteile |
| Wasser | 50,0 Gewichtsteile |

Hautpflegecreme in Gelform gegen Akne

| | |
|---|---|
| Cetylstearylalkohol mit circa 30 Mol Ethylenoxid | 14,0 Gewichtsteile |
| Polyol-Fettsäure-Ester (Cetiol HE(R)) | 20,0 Gewichtsteile |
| 2-Octyldodecanol | 5,0 Gewichtsteile |
| Produkt D | 3,0 Gewichtsteile |
| Wasser | 58,0 Gewichtsteile |

Hautpflegecreme gegen Akne

| | |
|---|---|
| Kolloiddisperses Gemisch aus 90 Teilen Cetylstearylalkohol und 10 Teilen Natrium-Cetylstearylsulfat | 15,0 Gewichtsteile |
| Reisstärke | 5,0 Gewichtsteile |
| Puderfarbe | 1,0 Gewichtsteile |
| Titandioxid | 2,0 Gewichtsteile |
| Produkt B | 5,0 Gewichtsteile |
| Wasser | 72,0 Gewichtsteile |

Hautpflegestift gegen Akne

| | |
|---|---|
| Cetylalkohol | 5,0 Gewichtsteile |
| Bienenwachs weiß | 30,0 Gewichtsteile |
| Walrat | 20,0 Gewichtsteile |
| 2-Octyldodecanol | 25,0 Gewichtsteile |
| Paraffinöl | 8,0 Gewichtsteile |
| Zinkoxid | 5,0 Gewichtsteile |
| Puderfarbe braun | 3,0 Gewichtsteile |
| Produkt E | 4,0 Gewichtsteile |

Hautpflegelotion gegen Akne

| | |
|---|---|
| Kolloiddisperses Gemisch aus 90 Teilen Cetylstearylalkohol und 10 Teilen Natrium-Cetylstearylsulfat | 3,0 Gewichtsteile |
| Ölsäuredecylester | 5,0 Gewichtsteile |
| Produkt H | 5,0 Gewichtsteile |
| Wasser | 87,0 Gewichtsteile |

Hautpflegeöl gegen Akne

| | |
|---|---|
| Ölsäuredecylester | 15,0 Gewichtsteile |
| 2-Octyldodecanol | 10,0 Gewichtsteile |
| Capryl/Caprinsäuretriglycerid | 30,0 Gewichtsteile |
| Paraffinöl | 35,0 Gewichtsteile |
| Produkt C | 10,0 Gewichtsteile |

/11

## Patentansprüche

1) Hautpflegemittel, gekennzeichnet durch einen Gehalt an arylsubstituierten Alkansäurederivaten der allgemeinen Formel

$$Ar - (X)_p - (CR_1R_2)_m - (CR_3R_4)_n - COR_5$$

in der Ar für einen gegebenenfalls substituierten Arylrest, X für Sauerstoff oder Schwefel, $R_1$ für Wasserstoff, einen niederen Alkylrest mit 1 bis 4 Kohlenstoffatomen oder einen gegebenenfalls substituierten Phenylrest, $R_2$ für Wasserstoff oder die OH-Gruppe, $R_3$ und $R_4$ unabhängig voneinander für Wasserstoff oder eine niedere Alkylgruppe mit 1 bis 4 Kohlenstoffatomen, $R_5$ für eine Alkoxygruppe mit 1 bis 10 Kohlenstoffatomen, eine $NH_2$-Gruppe, eine Mono- oder Dialkylaminogruppe, p für 0 oder 1, m für 1 oder 2 und n für 0, 1 oder 2 stehen neben üblichen Hilfs- und Trägerstoffen.

2) Hautpflegemittel nach Anspruch 1, dadurch gekennzeichnet, daß sie die arylsubstituierten Alkansäurederivate in einer Menge von 0,5 bis 30 Gewichtsprozent, vorzugsweise 3 bis 15 Gewichtsprozent, bezogen auf die gesamte Präparation, enthalten.

/12

**0039857**

Nummer der Anmeldung

Europäisches Patentamt

# EUROPÄISCHER RECHERCHENBERICHT

EP 81103325.7

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | GB - A - 916 242 (BOOTS PURE DRUG COMPANY LTD)<br>* Seiten 2,3,6; Beispiel 21 *<br>-- | 1,2 | A 61 K 7/48<br>A 61 K 7/32<br>A 61 K 31/215<br>A 61 K 31/165<br>A 61 K 31/085<br>A 61 K 31/10 |
| X | US - A - 4 105 782 (YU, VAN SCOTT)<br>* Zusammenfassung; Spalten 1,2*<br>-- | 1,2 | |
| X | US - A - 4 105 783 (YU, VAN SCOTT)<br>* Zusammenfassung; Spalten 1,2*<br>-- | 1,2 | |
| | US - A - 4 009 283 (HERR, JOHNSON)<br>* Spalten 1,2 *<br>-- | 1 | RECHERCHIERTE SACHGEBIETE (Int Cl.³) |
| | US - A 4 048 332 (ADAMS, ARMITAGE, NICHOLSON)<br>* Seite 1 *<br>-- | 1,2 | A 61 K 7/00<br>A 61 K 9/00<br>A 61 K 31/00<br>A 01 N 37/00<br>A 01 N 39/00<br>C 07 C 59/00<br>C 07 C 69/00<br>C 07 C 103/00 |
| | US - A 3 652 762 (SALLMANN, PFISTER)<br>* Spalten 1,6,7 *<br>-- | 1,2 | |
| | US - A - 4 048 330 (FRIED, HARRISON)<br>* Zusammenfassung; Spalten 1-3*<br>-- | 1 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | GB - A - 859 546 (HELLBAUM)<br>* Seiten 1-3 *<br>-- | 1 | |
| | GB - A - 971 700 (BOOTS PURE DRUG COMPANY LTD)<br>* Seiten 1,2,6,10 *<br>-- | 1 | |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| WIEN | 29-07-1981 | MAZZUCCO |

Europäisches Patentamt

**EUROPÄISCHER RECHERCHENBERICHT**

0039857

Nummer der Anmeldung

EP 81103325.7

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| | US - A - 3 993 763 (CARNEY, DE STEVENS)<br>* Zusammenfassung; Spalten 1, 2,16 *<br>---- | 1,2 | |
| | | | RECHERCHIERTE SACHGEBIETE (Int. Cl.³) |

EPA Form 1503.2 06.78